# EUROPEAN PATENT APPLICATION

(11) **EP 2 607 376 A1**
(43) Date of publication of application: **26.06.2013**
(21) Application number: 11195132.3
(22) Date of filing: 22.12.2011
(51) Int. Cl.: C07K 14/415

(54) **Hypoallergenic allergen derivatives of Pru p 3 for immunotherapy of IgE-mediated peach allergy**

(71) Applicant: BIOMAY AG, 1090 Wien (AT)
(72) Inventor: Linhart, Birgit, 3610 Weissenkirchen (AT); Gstöttner, Antonia, 4840 Vöcklabruck (AT); Valenta, Rudolf, 2604 Theresienfeld (AT); Papadopoulos, Nikolaos, 15561 Holargos (GR); Mari, Adriano, 04100 Latina (IT); Gamez, Cristina, 28022 Madrid (ES); Swoboda, Ines, 1020 Wien (AT)
(74) Representative: Keller, Günter

(57) **Abstract**

The major peach allergen Pru p 3 is responsible for allergies occurring mainly in the Mediterranean area. Hypoallergenic derivatives of Pru p 3 are disclosed which can be used as a vaccine in the treatment of allergic patients.

SEQUENCE LISTING

<110>  Biomay AG

<120>  Hypoallergenic allergen derivatives of Pru p 3 for immunotherapy
       of IgE - mediated peach allergy

<130>  Pru p 3

<160>  6

<170>  PatentIn version 3.5

<210>  1
<211>  98
<212>  PRT
<213>  Peach

<400>  1

Met Ile Thr Cys Gly Gln Val Ser Ser Ser Leu Ala Pro Cys Ile Pro
1               5                   10                  15

Tyr Val Arg Gly Gly Gly Ala Val Pro Pro Ala Cys Cys Asn Gly Ile
            20                  25                  30

Arg Asn Val Asn Asn Leu Ala Arg Thr Thr Pro Asp Arg Gln Ala Ala
        35                  40                  45

Cys Asn Cys Leu Lys Gln Leu Ser Ala Ser Val Pro Gly Val Asn Pro
    50                  55                  60

Asn Asn Ala Ala Ala Leu Pro Gly Lys Cys Gly Val Ser Ile Pro Tyr
65                  70                  75                  80

Lys Ile Ser Ala Ser Thr Asn Cys Ala Thr Val Lys His His His His
                85                  90                  95

His His

<210>  2
<211>  98
<212>  PRT
<213>  artificial sequence

<220>
<223>  derivative

<400>  2

Met Ile Thr Ser Gly Gln Val Ser Ser Ser Leu Ala Pro Ser Ile Pro
1               5                   10                  15

Tyr Val Arg Gly Gly Gly Ala Val Pro Pro Ala Ser Ser Asn Gly Ile
            20                  25                  30

Arg Asn Val Asn Asn Leu Ala Arg Thr Thr Pro Asp Arg Gln Ala Ala
        35                  40                  45

Ser Asn Ser Leu Lys Gln Leu Ser Ala Ser Val Pro Gly Val Asn Pro
    50                  55                  60

Asn Asn Ala Ala Ala Leu Pro Gly Lys Ser Gly Val Ser Ile Pro Tyr
65                  70                  75                  80

Lys Ile Ser Ala Ser Thr Asn Ser Ala Thr Val Lys His His His His
                85                  90                  95

His His

<210>  3
<211>  280
<212>  PRT
<213>  artificial sequence

<220>
<223>  derivative

<400>  3

Met Ile Thr Ser Gly Gln Val Ser Ser Ser Leu Ala Pro Ser Ile Pro
1               5                   10                  15

Tyr Val Arg Gly Gly Gly Ala Val Pro Pro Ala Ser Ser Asn Gly Ile
            20                  25                  30

Arg Asn Val Asn Asn Leu Ala Arg Thr Thr Pro Asp Arg Gln Ala Ala
        35                  40                  45

Ser Asn Ser Leu Lys Gln Leu Ser Ala Ser Val Pro Gly Val Asn Pro
    50                  55                  60

Asn Asn Ala Ala Ala Leu Pro Gly Lys Ser Gly Val Ser Ile Pro Tyr
65                  70                  75                  80

Lys Ile Ser Ala Ser Thr Asn Ser Ala Thr Val Lys Ile Thr Ser Gly
                85                  90                  95

Gln Val Ser Ser Ser Leu Ala Pro Ser Ile Pro Tyr Val Arg Gly Gly
            100                 105                 110

Gly Ala Val Pro Pro Ala Ser Ser Asn Gly Ile Arg Asn Val Asn Asn
        115                 120                 125

Leu Ala Arg Thr Thr Pro Asp Arg Gln Ala Ala Ser Asn Ser Leu Lys
    130                 135                 140

Gln Leu Ser Ala Ser Val Pro Gly Val Asn Pro Asn Asn Ala Ala Ala
145                 150                 155                 160

Leu Pro Gly Lys Ser Gly Val Ser Ile Pro Tyr Lys Ile Ser Ala Ser
                165                 170                 175

Thr Asn Ser Ala Thr Val Lys Ile Thr Ser Gly Gln Val Ser Ser Ser
            180                 185                 190

Leu Ala Pro Ser Ile Pro Tyr Val Arg Gly Gly Gly Ala Val Pro Pro
        195                 200                 205

Ala Ser Ser Asn Gly Ile Arg Asn Val Asn Asn Leu Ala Arg Thr Thr
    210                 215                 220

Pro Asp Arg Gln Ala Ala Ser Asn Ser Leu Lys Gln Leu Ser Ala Ser
225                 230                 235                 240

Val Pro Gly Val Asn Pro Asn Asn Ala Ala Ala Leu Pro Gly Lys Ser
                245                 250                 255

Gly Val Ser Ile Pro Tyr Lys Ile Ser Ala Ser Thr Asn Ser Ala Thr
            260                 265                 270

Val Lys His His His His His His
        275                 280

<210>  4
<211>  280
<212>  PRT
<213>  artificial sequence

<220>
<223>  derivative

<400>  4

Met Ile Arg Asn Val Asn Asn Leu Ala Arg Thr Thr Pro Asp Arg Gln
1               5                   10                  15

Ala Ala Cys Asn Cys Leu Lys Gln Leu Ser Ala Ser Val Ile Thr Cys
            20                  25                  30

Gly Gln Val Ser Ser Ser Leu Ala Pro Cys Ile Pro Tyr Val Arg Gly
        35                  40                  45

Gly Gly Ala Val Pro Pro Ala Cys Cys Asn Gly Pro Gly Val Asn Pro
    50                  55                  60

Asn Asn Ala Ala Ala Leu Pro Gly Lys Cys Gly Val Ser Ile Pro Tyr
65                  70                  75                  80

Lys Ile Ser Ala Ser Thr Asn Cys Ala Thr Val Lys Ile Arg Asn Val
                85                  90                  95

Asn Asn Leu Ala Arg Thr Thr Pro Asp Arg Gln Ala Ala Cys Asn Cys
            100                 105                 110

Leu Lys Gln Leu Ser Ala Ser Val Ile Thr Cys Gly Gln Val Ser Ser
        115                 120                 125

Ser Leu Ala Pro Cys Ile Pro Tyr Val Arg Gly Gly Gly Ala Val Pro
    130                 135                 140

Pro Ala Cys Cys Asn Gly Pro Gly Val Asn Pro Asn Asn Ala Ala Ala
145                 150                 155                 160

Leu Pro Gly Lys Cys Gly Val Ser Ile Pro Tyr Lys Ile Ser Ala Ser
                165                 170                 175

Thr Asn Cys Ala Thr Val Lys Ile Arg Asn Val Asn Asn Leu Ala Arg
            180                 185                 190

Thr Thr Pro Asp Arg Gln Ala Ala Cys Asn Cys Leu Lys Gln Leu Ser
        195                 200                 205

Ala Ser Val Ile Thr Cys Gly Gln Val Ser Ser Ser Leu Ala Pro Cys
    210                 215                 220

Ile Pro Tyr Val Arg Gly Gly Gly Ala Val Pro Pro Ala Cys Cys Asn
225                 230                 235                 240

Gly Pro Gly Val Asn Pro Asn Asn Ala Ala Ala Leu Pro Gly Lys Cys
                245                 250                 255

Gly Val Ser Ile Pro Tyr Lys Ile Ser Ala Ser Thr Asn Cys Ala Thr
            260                 265                 270

Val Lys His His His His His His
        275                 280

<210>  5
<211>  280
<212>  PRT
<213>  artificial sequence

<220>
<223>  derivative

<400>  5

Met Ile Arg Asn Val Asn Asn Leu Ala Arg Thr Thr Pro Asp Arg Gln
1               5                   10                  15

Ala Ala Ser Asn Ser Leu Lys Gln Leu Ser Ala Ser Val Ile Thr Ser
            20                  25                  30

Gly Gln Val Ser Ser Ser Leu Ala Pro Ser Ile Pro Tyr Val Arg Gly
        35                  40                  45

Gly Gly Ala Val Pro Pro Ala Ser Ser Asn Gly Pro Gly Val Asn Pro
    50                  55                  60

Asn Asn Ala Ala Ala Leu Pro Gly Lys Ser Gly Val Ser Ile Pro Tyr
65                  70                  75                  80

Lys Ile Ser Ala Ser Thr Asn Ser Ala Thr Val Lys Ile Arg Asn Val
                85                  90                  95

Asn Asn Leu Ala Arg Thr Thr Pro Asp Arg Gln Ala Ala Ser Asn Ser
            100                 105                 110

Leu Lys Gln Leu Ser Ala Ser Val Ile Thr Ser Gly Gln Val Ser Ser
        115                 120                 125

Ser Leu Ala Pro Ser Ile Pro Tyr Val Arg Gly Gly Gly Ala Val Pro
    130                 135                 140

Pro Ala Ser Ser Asn Gly Pro Gly Val Asn Pro Asn Asn Ala Ala Ala
145                 150                 155                 160

Leu Pro Gly Lys Ser Gly Val Ser Ile Pro Tyr Lys Ile Ser Ala Ser
                165                 170                 175

Thr Asn Ser Ala Thr Val Lys Ile Arg Asn Val Asn Asn Leu Ala Arg
            180                 185                 190

Thr Thr Pro Asp Arg Gln Ala Ala Ser Asn Ser Leu Lys Gln Leu Ser
        195                 200                 205

Ala Ser Val Ile Thr Ser Gly Gln Val Ser Ser Ser Leu Ala Pro Ser
    210                 215                 220

Ile Pro Tyr Val Arg Gly Gly Gly Ala Val Pro Pro Ala Ser Ser Asn
225                 230                 235                 240

Gly Pro Gly Val Asn Pro Asn Asn Ala Ala Ala Leu Pro Gly Lys Ser
                245                 250                 255

Gly Val Ser Ile Pro Tyr Lys Ile Ser Ala Ser Thr Asn Ser Ala Thr
            260                 265                 270

Val Lys His His His His His His
        275                 280

<210>  6
<211>  98
<212>  PRT
<213>  Prunus persica

<400>  6

Met Ile Thr Cys Gly Gln Val Ser Ser Ser Leu Ala Pro Cys Ile Pro

1               5                   10                  15

Tyr Val Arg Gly Gly Gly Ala Val Pro Pro Ala Cys Cys Asn Gly Ile
            20                  25                  30

Arg Asn Val Asn Asn Leu Ala Arg Thr Thr Pro Asp Arg Gln Ala Ala
        35                  40                  45

Cys Asn Cys Leu Lys Gln Leu Ser Ala Ser Val Pro Gly Val Asn Pro
    50                  55                  60

Asn Asn Ala Ala Ala Leu Pro Gly Lys Cys Gly Val Ser Ile Pro Tyr
65                  70                  75                  80

Lys Ile Ser Ala Ser Thr Asn Cys Ala Thr Val Lys His His His His
                85                  90                  95

His His

## Description

The present invention relates to the field of immunotherapy against IgE-mediated food allergy, in particular allergy to peach. More specifically the invention relates to the design of new recombinant hypoallergenic vaccines against peach allergy.

Allergens are, by definition, antigens that can elicit specific IgE responses in genetically susceptible individuals. In general, allergens are subdivided by route of exposure and source. Such allergens include aeroallergens (pollens, mould spores, animal dander, faecal materials excreted by mice and cockroaches), food allergens, stinging insects like bees or wasps, pharmaceuticals and latex.

The adaptive immune response is a critical component of host defence against infection and is essential for normal health. Unfortunately, adaptive immune responses are also sometimes elicited by antigens not associated with harmful (e.g. infectious) agents and this can cause serious diseases. Such harmful immune reactions are generally known as hypersensitivity reactions which are made in response to inherently harmless environmental antigens derived from pollen, food and drugs (allergens).

Hypersensitivity reactions were classified into four types, namely Type I, Type II, Type III and Type IV. Allergy is the commonest type of hypersensitivity which is often equated with Type I hypersensitivity reactions which are immediate-type hypersensitivity reactions mediated by IgE antibodies. In the majority of allergies such as those to food, reactions occur because the individual has become sensitized to a per se innocuous antigen, the allergen, by producing IgE antibodies against it. Subsequent exposure to the allergen triggers the activation of IgE-binding cells, including mast cells and basophils in the exposed tissue leading to a series of responses that are characteristic of allergy and are commonly known as allergic reactions.

The most severe manifestation of IgE-mediated allergy is life-threatening anaphylaxis, which occurs after systemic uptake of allergens, e.g. in drug allergy, insect venom and food allergy. Food allergy represents the most frequent cause of systemic anaphylaxis in industrialized countries.

The present invention relates to allergy against peach and products containing peach derivatives which occurs frequently in mediterranean countries wherein peaches form a substantial part of the consumed fruits. One of the major antigens responsible for an allergy against peach and peach-containing products is Pru p 3.

Pru p 3, a small protein (9 kDa) from peach, is involved in plant defense mechanisms against pathogens. It is very resistant to heat treatment and pepsin digestion and represents a severe food-derived panallergen. It belongs to the family of nonspecific lipid transfer proteins (LTPs) present in fruits, vegetables, nuts, pollen, and latex, which were identified as important elicitors of IgE-mediated food hypersensitivity reactions. Due to structural similarities LTPs from different allergen sources may represent cross-reactive allergens. The lipid transfer protein present in peach (Pru p 3) was identified as the predominant LTP regarding IgE recognition of allergic patients. It contains eight conserved cysteines forming four disulphide bridges which stabilize the 3-dimensional structure with four helices and a C-terminal tail.

Allergen-specific immunotherapy (SIT) is currently the only antigen-specific and disease-modulating therapy for IgE-mediated allergy (Larche, Akdis & Valenta, Nat.Rev.Immunol. 2006; 6: 761-71). It is based on the administration of the disease-causing allergens with the aim to induce allergen-specific protective IgG antibodies and to modulate allergen-specific T cell responses. However, the administration of allergens in the course of SIT may induce allergic side effects of which the most severe and life-threatening reactions are caused by IgE-mediated mast cell and basophil degranulation. In order to reduce these side effects it has been suggested to produce recombinant hypoallergenic allergen-derivatives with strongly reduced or even abolished IgE reactivity for SIT (Valenta, Nat.Rev.lmmunol. 2002; 2: 446-53).

An increasing body of evidence published by different authors has shown that the induction of antibodies of IgG isotype may be crucial for successful allergen specific immunotherapy. It is believed that such blocking IgG antibodies, which bind allergens, will prevent IgE production or suppress IgE-mediated presentation of the allergen, thus reducing T cell proliferation and the release of cytokines (Linhart and Valenta, Vaccine 2011, in press). The hypoallergenic derivatives of Pru p 3 as described herein are designed to elicit after administration to allergic patients such protective IgG antibodies.

Several strategies for the production of hypoallergens have been developed in the past. They include the introduction of point mutations into the allergen sequence, fragmentation and production of mosaic antigens consisting of recombined allergen fragments.

In fact attempts have been made to produce hypoallergenic Pru p 3 derivatives by several strategies in the past. However, reduction and alkylation of Pru p 3 led to a reduction of IgE binding, but the "hypoallergens" described by Toda et al. [J.Allergy Clin.Immunol. (2011), in press], did not induce Pru p 3-specific IgG antibodies upon immunization and therefore will not induce protective IgG antibodies upon SIT.

The allergen preparation as used by Toda et al. was native Pru p 3 purified from freshly prepared peach peel extract. The purified native Pru p 3 was reduced and alkylated by adding iodoacetamide in order to disrupt the tertiary structure of the allergen.

Diaz-Perales et al. [Clin.Exp.All. (2002) 32, pp 87-92] described the cloning and heterologous expression of the major allergens from peach and apple belonging to the lipid protein family. In this publication it is disclosed that Pru p 3 has clinical relevance in a population of Spanish patients with allergy to peach fruits. However, this work does not disclose recombinant hypoallergens of Pru p 3 with reduced IgE reactivity which would be suitable for SIT with reduced side effects.

García-Casado et al. [J.Allergy Clin.Immunol. (2003) pp 599-605] identified IgE-binding epitopes on the major peach allergen Pru p 3. In this publication three sequence regions of Pru p 3, namely 11 to 25, 31 to 45 and 71 to 80 have been identified as major IgE epitopes.

García-Casado et al., induced point mutations but achieved only a low (approx. five-fold) reduced IgE reactivity. A five-fold reduction of IgE reactivity and allergenic activity is considered to be insufficient to yield a safe hypoallergenic vaccine (Linhart and Valenta, Vaccine 2011, in press). Furthermore, they identified several small (less than 20 aa) peptide IgE epitopes so that fragmentation or mosaic approaches appeared not suitable for the generation of hypoallergenic Pru p 3 derivatives.

It is an object of the present invention to provide suitable modified allergen derivatives of Pru p 3 which show a more than 10-fold reduction of IgE reactivity or allergenic activity, induce robust allergen-specific IgG responses and can be used for the treatment of allergy.

The aim of this work was to design hypoallergenic derivatives of Pru p 3 with strongly reduced (more than 10-fold) IgE reactivity and allergenic activity which induce upon immunization Pru p 3 specific IgG antibodies for immunotherapy of peach allergy. The hypoallergenic derivatives as disclosed herein were produced recombinantly. Therefore, the derivatives can be exactly reproduced.

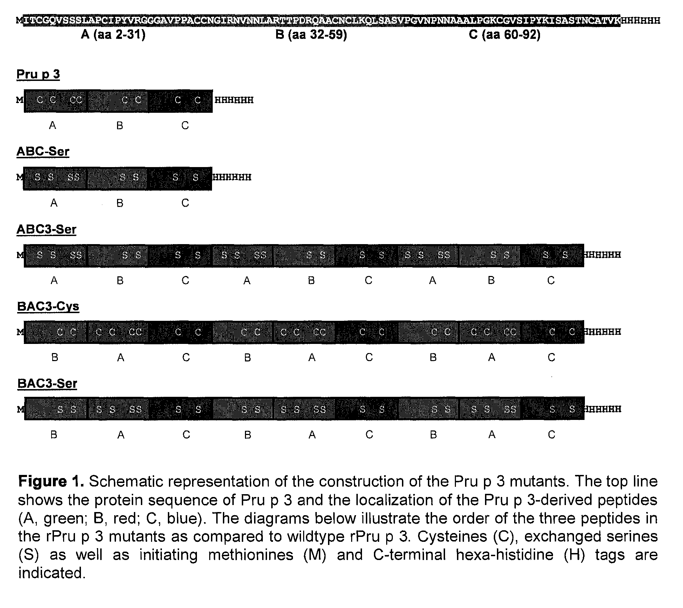

For a better understanding of the present invention, we refer to Figure 1. On the top of Figure 1, the sequence of Pru p 3 (SEQ ID NO: 6) is shown whereby three parts (A, B and C) of the protein are shown. Part A comprises amino acids 10-20 and ranges preferably from amino acid 2-31. Part B comprises amino acids 40-50 and preferably ranges from amino acid 32-59. Part C comprises amino acids 70-80 and preferably ranges from amino acid 60-92. The sequence has at the C-terminus a polyhistidine-tag just to facilitate the purification of the proteins.

In a preferred embodiment the hypoallergenic derivative of the present invention comprises stretches consisting of at least 10 amino acids, preferably 15 and more preferred at least 20 amino acids selected from the parts A, B and C of the antigen Pru p 3.

The amino acid sequence derived from part A must include the range between amino acid 10 and 20. The part of the hypoallergenic derivative derived from part A of Pru p 3 may, however, be derived from any part of fragment A which consists of amino acids 2-31 provided that the sequence between amino acid 10 and 20 is contained within this part.

Part B of the allergen Pru p 3 ranges from amino acids 32-59. The hypoallergenic derivative of the present invention comprises a stretch of at least 10 amino acids which must comprise amino acids 40-50. According to the present invention the hypoallergenic derivative does therefore include any peptide ranging from amino acid 32 to 50 or alternatively from amino acid 40 to 59.

The hypoallergenic derivative of Pru p 3 comprises also a portion derived from part C of the allergen. Part C ranges from amino acid 60-92. The derivative of part C contained within the hypoallergenic derivative according to the present invention must contain amino acids 70-80. Therefore, the hypoallergenic derivative of Pru p 3 comprises any peptide ranging from amino acids 60-80 to any peptide extending from amino acid 70-92.

In a particular preferred embodiment each part A, B and C of the hypoallergenic derivative of Pru p 3 consists of at least 20 amino acids.

The next line in Figure 1 under the heading Pru p 3 shows schematically the sequence wherein location of the cysteine residues is shown. According to the present invention at least one of the cysteine residues in each of parts A, B and C is replaced by the amino acid serin. In a more preferred embodiment of the present invention at least two cysteine residues in each part A, B and C are replaced by serin and in a particular preferred embodiment all cysteines are replaced by serin residues. The sequence of such a preferred embodiment is shown in the next line of Figure 1 under the heading ABC-Ser.

In another preferred embodiment of the present invention the modified derivative of Pru p 3 wherein the cysteine residues have been replaced by serin residues are used as multimers whereby two or preferably three copies of the derivative of Pru p 3 have been combined to an oligomeric derivative. This is schematically shown in Figure 1 under the heading ABC3-Ser. Although not shown in Figure 1, the oligomeric hypoallergenic derivatives of Pru p 3 may retain some cysteine residues provided that in each part A, B and C at least one cysteine is replaced by a serin.

In another embodiment of the present invention the order of the parts A, B and C has been changed. In Figure 1 under the heading BAC-Cys such a modified molecule is schematically shown whereby the cysteine residues have not been replaced by serin. The cystein residues are preferably replaced by serin. It is, however, also possible to use other amino acids instead of serin. Such amino acids do preferably not have charged side chains.

The line under the heading BAC3-Ser in Figure 1 shows the same molecule wherein the cysteine residues have been replaced by serin residues according to the invention.

By replacing at least several of the cysteine residues by serin residues the tertiary structure of the molecule is changed. It is understood that the epitopes to which IgE antibodies can bind are changed. Therefore the derivatives of the present invention are hypoallergenic. This means that upon administration in the form of a vaccination or hyposensibilisation the risk of undesired allergic side reactions such as anaphylactic shock is substantially reduced. Nevertheless, the hypoallergenic derivatives of the present invention do produce blocking IgG antibodies which are very helpful for counteracting allergic reactions.

In a preferred embodiment of the present invention the hypoallergenic derivative of Pru p 3 comprises at least three parts of the antigen Pru p 3 whereby part A of Pru p 3 comprises amino acids 10 to 20, part B of Pru p 3 comprises amino acids 40 to 50 and part C comprises amino acids 70 to 80 and wherein in each of parts A, B and C at least one cysteine residue is replaced by a serin residue. In each of said parts A, B and C at least one, preferably two and most preferably all cysteines have been replaced by serin.

In another preferred embodiment the hypoallergenic derivative of Pru p 3 contains at least one part selected from parts A, B and C twice in the molecule. It is more preferred that a hypoallergenic derivative of Pru p 3 contains each of parts A, B and C at least twice or even more preferred three times in the hybrid allergen molecule.

Preferably the natural order of the parts A-B-C is changed to the order B-A-C, or B-C-A or C-B-A or C-A-B or A-C-B. Combinations thereof are provided when each of parts A, B, C is contained twice or three times in the molecule.

The hypoallergenic derivatives of Pru p 3 as disclosed in the present application can preferably be used as medicament for the treatment of allergy. Such medicament is preferably used as a vaccine which elicits the production of neutralizing IgG antibodies directed against the native allergen Pru p 3. The medicament of the present invention is preferably used in a hyposensibilisation treatment of patients suffering from allergy against peaches and fruits related thereto. The medical treatment may be effective also in allergies against related fruits like apricots.

In the course of a hyposensibilisation the hypoallergenic derivative of Pru p 3 as disclosed in the present application is administered to the patient preferably several times (two times up to seven times) at intervals ranging between two weeks to three months between each application. In order to increase the efficacy of the vaccine as disclosed herein it may be used together with an adjuvant which is admitted for administration to human beings. Considering the results of previous work it was unexpected to find that exchange of the cysteine residues lead to a hypoallergenic Pru p 3 derivative because Garcia-Casado et al. have shown that the introduction of point mutations does not sufficiently reduce the IgE reactivity of Pru p 3. Furthermore, they showed, that Pru p 3 contains overall sequential IgE epitopes which are not affected by the change of cysteins to serins. Finally it has been shown by Toda et al. that a reduction of the protein which targets the cysteins leads to a non-immunogenic protein whereas the derivatives enclosed in this application induced Pru p 3-specific IgG. Furthermore, it was unexpected that mosaic versions of Pru p 3 and their trimeric forms remained non-IgE reactive and hypoallergenic but induced allergen-specific IgG.

It was surprising that the mosaic version of Pru p 3 exhibited more than 10-fold reduced IgE reactivity and allergenic activity because it has been shown by Garcia-Casado et al. that Pru p 3 contains several small sequential IgE epitopes, which should not be destroyed by the mosaic approach. An earlier described hypoallergenic Bet v 1 trimer was hypoallergenic but showed comparable IgE reactivity as the Bet v 1 allergen (Vrtala S et al, FASEB J. 2001, 15: 2045-7; Campana R et al, Mol. Immunol. 2011, 48: 431-41). By contrast, the trimeric versions of Pru p 3 showed a strongly reduced (more than 10-fold) IgE-reactivity. The features of the disclosed derivatives which are suitable as hypoallergens for SIT of peach allergy were therefore unexpected.

Preferred embodiments of the present invention are shown in the figures.

**Figure 1** shows schematically the construction of the Pru p 3 mutants. The top line shows the protein sequence of Pru p 3 (corresponding to Seq ID No. 6) and the localization of the three parts A, B and C. The next line under the heading Pru p 3 shows the three parts whereby the location of the cysteines in the parts has been highlighted.

In the next line the same construction is shown under the heading ABC-Ser, whereby all cysteine (C) residues have been replaced by serin (S) residues. It is evident that also such embodiments are encompassed by the present invention wherein only a part of the cysteine residues has been replaced by serin residues provided that in each part A, B and C at least one cysteine residue has been replaced by serin. Under the heading ABC3-Ser a construct is shown schematically wherein the modified derivative of Pru p 3 is shown as a trimer.

Under the heading BAC3-Cys an embodiment is shown whereby the order of the parts A, B and C has been changed to BAC. In this construct, the cysteines (C) are shown. According to the invention, however, at least one of the cystein residues in each of the parts A, B and C has to be replaced by a serin (not shown in Figure 1).

BAC3-Ser shows a multimeric derivative whereby the order of the parts has been changed.

The sequences as shown contain at the C-terminus a polyhistidine tag which is helpful for the production of the polypeptide but do not affect the properties of the protein. Such histidine tags are then normally removed before application of the polypeptide as vaccine.

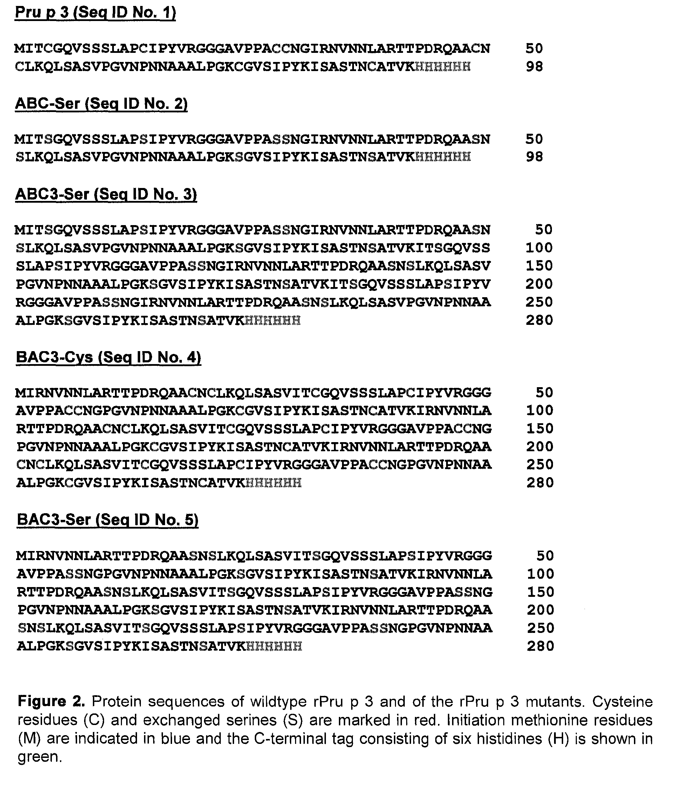

**Figure 2** shows the protein sequences of wildtype Pru p 3 and the sequences of preferred embodiments of the present invention whereby the cysteines have been replaced by serin.

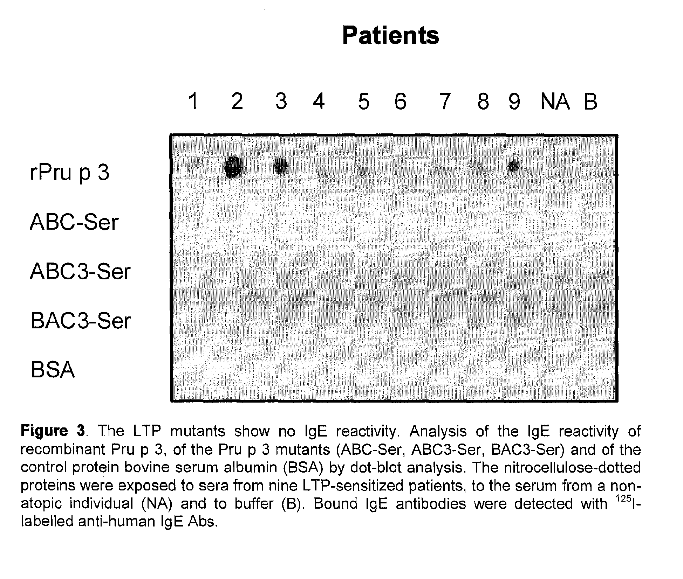

**Figure 3** shows certain results of the experiments performed with the present invention. The IgE reactivity of several sera (1-9) has been tested with wildtype recombinantly produced Pru p 3. In the first line it can be seen, that recombinant Pru p 3 shows IgE reactivity with sera from peach allergic patients. In the lines below, hypoallergenic derivatives according to the present invention, namely ABC-Ser, ABC3-Ser and BAC3-Ser,

have been tested. Those molecules do not show any reactivity with the sera from allergic patients. The IgE-reactivity and allergenic activity is therefore more than 10-fold reduced. The last line is bovine serum albumin (BSA) which serves as control.

### Example 1

In order to generate hypoallergenic mutants of Pru p 3, the lipid transfer protein (LTP) from peach, with reduced allergenic activity and preserved immunogenicity three fragments (referred to as A, B, and C) based on the Pru p 3 aa sequence were designed (Figure 1). DNA sequences encoding wildtype Pru p 3 (accession number Q9LED1) and the Pru p 3 mutants with C-terminal hexa-histidine tags were codon optimized for expression in *Escherichia coli* and synthesized by GenScript (Piscataway, NJ). The synthetic genes encoded the following proteins which are shown in Figure 2:
1. Pru p 3 (Seq ID No. 1)
2. A Pru p 3 mutant based on the Pru p 3 amino acid sequence with 8 point mutations replacing cysteines by serins (referred to as ABC-Ser, Seq ID No. 2)
3. A Pru p 3 mutant representing a trimeric form of the ABC-Ser protein (referred to as ABC3-Ser, Seq ID No. 3)
4. A Pru p 3 mutant representing a mosaic molecule consisting of the Pru p 3 derived fragments A, B, and C in the order B-A-C in a trimeric form (referred to as BAC3-Cys, Seq ID No. 4)
5. A Pru p 3 mutant based on the amino acid sequence of BAC3-Cys with 24 point mutations replacing cysteines by serins (referred to as BAC3-Ser, Seq ID No. 5).

### Example 2

The synthetic genes were cloned into the Ndel and EcoRI restriction sites of the bacterial expression vector pET-27b (Novagen, Madison, WI). Expression of the recombinant proteins was induced in the *E*. *coli* strain BL21 (DE3) by addition of IPTG and the proteins were purified under native or denaturing conditions by nickel affinity chromatography (Qiagen, Hilden, Germany). The biochemical features of the recombinant proteins are shown in Table 1.

**Table 1: Characteristics of rPru p 3 and the rPru p 3 mutants.**

| **Proteins** | **Number of amino acids** | **Molecular mass (Da)** | **Isoelectric point** |
|---|---|---|---|
| **rPru p 3** | 98 | 10097.6 | 9.3 |
| **ABC-Ser** | 98 | 9969.1 | 11.1 |
| **ABC3-Ser** | 280 | 27963.2 | 11.5 |
| **BAC3-Cys** | 280 | 28348.6 | 9.4 |
| **BAC3-Ser** | 280 | 27963.2 | 11.5 |

### Example 3

IgE reactivity of rPru p 3 and the three rPru p 3 mutants ABC-Ser, ABC3-Ser and BAC3-Ser was evaluated by dot-blot analysis (Figure 3). Nitrocellulose-dotted rPru p 3, rPru p 3 mutants and the control protein bovine serum albumin (BSA) were exposed to sera from LTP-sensitized patients, to the serum from a non-atopic individual and to buffer. Bound IgE antibodies were detected with ¹²⁵I-labelled anti-human IgE antibodies. As exemplified in Figure 3 with sera from nine LTP-sensitized patients, wildtype rPru p 3 was recognized by patients' IgE antibodies, whereas no serum reacted with any of the three mutants. Each of the disclosed hypoallergenic rPru p 3 derivatives induced Pru p 3-specific IgG antibodies upon immunization of rabbits. Since an at least 10-fold reduced reactivity with IgE antibodies was detected and the derivatives induced Pru p 3-specific IgG, the molecules may be used as vaccines without the risk of inducing unwanted side effects such as anaphylactic shock.

### Example 4

Rabbits were immunized with the recombinant purified Pru p 3 derivatives described in Example 2 in order to study their immunogenicity and their ability to induce an antibody response specific for the natural Pru p 3 allergen.

Rabbits were immunized according to standard procedure and the Pru p 3 derivatives according to the invention were compared with controls. Serum samples were taken at various time points and the formation of IgG antibodies against the allergens was measured. The derivatives were shown to induce a robust Pru p 3-specific IgG antibody response.

## Claims

1. Hypoallergenic derivative of Pru p 3 **characterized in that** it comprises at least three parts of the antigen Pru p 3 whereby part A of Pru p 3 comprises amino acids 10 to 20, part B of Pru p 3 comprises amino acids 40 to 50 and part C comprises amino acids 70 to 80 and wherein in each of parts A, B and C at least one cysteine residue is replaced by a serin residue.

2. Hypoallergenic derivative of Pru p 3 according to claim 1, **characterized in that** it comprises Seq ID No. 2.

3. Hypoallergenic derivative of Pru p 3 according to claim 1 **characterized in that** it contains at least one part selected from parts A, B and C twice in the molecule.

4. Hypoallergenic derivative of Pru p 3 according to claim 1 **characterized in that** it contains each of parts A, B and C twice.

5. Hypoallergenic derivative of Pru p 3 according to claim 1 **characterized in that** it contains each of parts A, B and C three times in the hybrid allergen molecule.

6. Hypoallergenic derivative of Pru p 3 according to claim 5, **characterized in that** it comprises Seq ID No. 3.

7. Hypoallergenic derivative of Pru p 3 according to any of claims 1, 3, 4 and 5 **characterized in that** the natural order of the parts A-B-C is changed to the order B-A-C, or B-C-A or C-B-A or C-A-B or A-C-B and combinations thereof when each of parts A, B, C is contained twice or three times in the molecule.

8. Hypoallergenic derivative of Pru p 3 according to claim 7, **characterized in that** it comprises SEQ ID NO:5.

9. Medicament for the treatment of allergy **characterized in that** it comprises a hypoallergenic derivative of Pru p 3 according to any one of claims 1 to 8.

10. Hypoallergenic derivative of Pru p 3 according to any of claims 1 to 8 for use as vaccine.

11. Hypoallergenic derivative of Pru p 3 according to claim 10 for use as vaccine for the production of neutralizing IgG antibodies.
